# FASCICULE DE BREVET EUROPEEN

(11) **EP 1 047 377 B1**
(45) Date de publication et mention de la délivrance du brevet: **27.06.2001**
(21) Numéro de dépôt: 98962518.1
(22) Date de dépôt: 18.12.1998
(51) Int. Cl.: A61K 7/13

(54) **COMPOSITION TINCTORIALE CONTENANT UNE LACCASE ET PROCEDES DE TEINTURE DES FIBRES KERATINIQUES LA METTANT EN OEUVRE**
LACCASE ENTHALTENDES FAERBEMITTEL UND FAERBUNG VON KERATINFASERN MIT DIESEM MITTEL
DYEING COMPOSITION CONTAINING A LACCASE AND KERATINOUS FIBRE DYEING METHODS USING SAME

(30) Priorité: 13.01.1998 FR 9800254
(43) Date de publication de la demande: 02.11.2000
(73) Titulaire: L'OREAL, 75008 Paris (FR)
(72) Inventeur: LANG, Gérard, F-95390 Saint Prix (FR); COTTERET, Jean, F-78480 Verneuil/Seine (FR)
(74) Mandataire: Miszputen, Laurent
(86) Numéro de dépôt international: FR9802794
(87) Numéro de publication internationale: WO9936035

(56) Documents cités:
- EP-A- 0 504 005
- EP-A- 0 557 203
- EP-A- 0 673 641
- FR-A- 2 694 018

## Description

La présente invention a trait à une composition tinctoriale des fibres kératiniques comprenant dans un support approprié pour la teinture des fibres kératiniques au moins une enzyme de type laccase, au moins un polymère substantif cationique ou amphotère particulier et au moins un colorant d'oxydation, ainsi que ses utilisations pour la teinture des fibres kératiniques en particulier des cheveux humains.

Il est connu de teindre les fibres kératiniques et en particulier les cheveux humains avec des compositions tinctoriales contenant des précurseurs de coloration d'oxydation, en particulier des ortho ou paraphénylènediamines, des ortho ou paraaminophénols, des bases hétérocycliques, appelés généralement bases d'oxydation. Les précurseurs de coloration d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologigue, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé dans le brevet US 3251742, les demandes de brevet FR-A-2 112 549, FR-A-2 694 018, EP-A-0 504 005, WO95/07988, WO95/33836, WO95/33837, WO96/00290, WO97/19998 et WO97/19999 de teindre les fibres kératiniques avec des compositions comprenant au moins un colorant d'oxydation en association avec des enzymes du type laccase ; lesdites compositions étant mises en contact avec l'oxygène de l'air. Ces formulations de teinture, bien qu'étant mises en oeuvre dans des conditions n'entraînant pas une dégradation des fibres kératiniques comparable à celle engendrée par les teintures réalisées en présence de peroxyde d'hydrogène, conduisent à des colorations encore insuffisantes à la fois sur le plan de l'homogénéité de la couleur répartie le long de la fibre (« unisson »), sur le plan de la chromaticité (luminosité) et de la puissance tinctoriale.

La présente invention a pour but de résoudre les problèmes évoqués ci-dessus La Demanderesse a découvert de façon surprenante de nouvelles compositions tinctoriales contenant au moins comme système oxydant une enzyme du type laccase et au moins un polymère substantif cationique ou amphotère particulier que l'on définira plus en détail ci-dessous, pouvant constituer en présence de colorant(s) d'oxydation (bases d'oxydation et/ou coupleurs), des formulations de teinture prêtes à l'emploi conduisant à des colorations plus homogènes, plus puissantes et plus chromatiques sans engendrer de dégradation significative, ni de décoloration des fibres kératiniques, peu sélectives et résistant bien aux diverses agressions que peuvent subir les cheveux.

Ces découvertes sont à la base de la présente invention.

La présente invention a donc pour premier objet une composition prête à l'emploi destinée à la teinture des fibres kératiniques, en particulier des fibres kératiniques humaines et plus particulièrement les cheveux humains, comprenant dans un support approprié pour la teinture,
(a) au moins une enzyme de type laccase ;
(b) au moins un polymère substantif cationique ou amphotère choisi dans le groupe formé par :
   **(i)** le Polyquaternium-24 ;
   **(ii)** les copolymères d'halogénure de diméthyldiallylammonium;
   **(iii)** les homopolymères et copolymères d'halogénure de méthacryloyloxyéthyltriméthylammonium ;
   **(iv)** les polymères polyammonium quaternaire choisis parmi ceux de formules (I), (II), et (III) définies dans la suite du texte;
   **(v)** les copolymères de vinylpyrrolidone à motifs cationiques ;
   **(vi)** les polysiloxanes cationiques.
(c) au moins un colorant d'oxydation.

La ou les laccases utilisées dans la composition tinctoriale prête à l'emploi conforme à l'invention peuvent notamment être choisies parmi les laccases d'origine végétale, d'origine animale, d'origine fongique (levures, moisissures, champignons) ou d'origine bactérienne, les organismes d'origine pouvant être mono ou pluricellulaires. Elles peuvent être obtenues par biotechnologie.

Parmi les laccases d'origine végétale utilisables selon l'invention, on peut citer les laccases produites par des végétaux effectuant la synthèse chlorophyllienne telles qu'indiquées dans la demande FR-A-2 694 018 comme celles que l'on retrouve dans les extraits des Anacardiacées tels que par exemple les extraits de Magnifera indica, Schinus molle ou Pleiogynium timoriense, dans les extraits des Podocarpacées, de Rosmarinus off., de Solanum tuberosum, d'Iris sp., de Coffea Sp. , de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.

Parmi les laccases d'origine fongique éventuellement obtenues par biotechnologie utilisables selon l'invention, on peut citer la ou les laccases issues de Polyporus versicolor, de Rhizoctonia praticola et de Rhus vernicifera comme indiquées dans les demandes FR-A-2 112 549 et EP-A-504005 ; celles décrites dans les demandes de brevet WO95/07988, WO95/33836, WO95/33837, WO96/00290, WO97/19998 et WO97/19999, dont le contenu fait partie intégrante de la présente description comme par exemple celles issues de Scytalidium, de Polyporus pinsitus, de Myceliophtora thermophila, de Rhizoctonia solani, de Pyricularia orizae, ou leurs variantes. On peut aussi citer celles issues de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor de Coriolus hirsutus, de Ceriporiopsis subvermispora, de Coprinus cinereus, de Panaeolus papilionaceus, de Panaeolus sphinctrinus, de Schizophyllum commune, de Dichomitius squalens et de leurs variantes.

On choisira plus préférentiellement les laccases d'origine fongique éventuellement obtenues par biotechnologie.

L'activité enzymatique des laccases de l'invention ayant la syringaldazine parmi leurs substrats peut être définie à partir de l'oxydation de la syringaldazine en condition aérobie. L'unité lacu correspond à la quantité d'enzyme catalysant la conversion de 1mmole de syringaldazine par minute à pH 5,5 à 30°C. L'unité u correspond à la quantité d'enzyme produisant un delta d'absorbance à 530 nm de 0.001 par minute en utilisant la syringaldazine comme substrat, à 30°C et à pH 6,5.
L'activité enzymatique des laccases de l'invention peut aussi être définie à partir de l'oxydation de la paraphénylènediamine. L'unité ulac correspond à la quantité d'enzyme produisant un delta d'absorbance à 496,5nm de 0,001 par minute en utilisant la paraphénylènediamine comme substrat (64 mM) à 30°C et à pH 5. Selon l'invention, on préfère déterminer l'activité enzymatique en unités ulac. Les quantités de laccase utilisées dans les compositions de l'invention varieront en fonction de la nature de la laccase choisie. De façon préférentielle, elles varieront de 0,5 à 2000 lacu, ou de 1000 à 4.10⁷ unités u, ou de 20 à 2.10⁶ unités ulac pour 100g de composition.

Le caractère substantif (c'est à dire l'aptitude au dépôt sur les cheveux) des polymères utilisés conformément à l'invention est classiquement déterminé au moyen du test décrit par Richard J. Crawford, Journal of the Society of Cosmetic Chemists, 1980, 31 - (5) - pages 273 à 278 (révélation par colorant acide Red 80).
Ces polymères substantifs sont notamment décrits dans la littérature dans la demande de brevet EP-A-0557203.

Le "Polyquaternium-24" est une définition du dictionnaire C.T.F.A. (5^{eme} édition, 1993) qui désigne un polymère d'ammonium quaternaire d'hydroxyéthylcellulose ayant réagi avec un époxyde substitué par un groupement lauryldiméthylammonium. Ce polymère est décrit dans la demande de brevet EP-A- 0 189 935 et commercialisé sous la dénomination "Quatrisoft LM 200" par la société Union Carbide.

Parmi les polymères substantifs du type copolymère d'halogénure de diméthyldiallylammonium utilisables selon l'invention, on peut citer en particulier :
- les copolymères de chlorure de diallyldiméthylammonium et d'acide acrylique comme celui de proportions (80/20 en poids) vendu sous la dénomination Merquat 280 par la société Calgon;
- les copolymères du chlorure de diméthyldiallylammonium et de l'acrylamide vendus sous les dénominations Merquat 550 et Merquat S par la société Merck.

Parmi les polymères substantifs du type polymère d'halogénure de méthacryloyloxyéthyltriméthylammonium utilisables selon l'invention, on peut citer en particulier les produits qui sont dénommés dans le dictionnaire CTFA (5ème édition, 1993) "Polyquaternium 37", "Polyquaternium 32" et "Polyquaternium 35", qui correspondent respectivement, en ce qui concerne le "Polyquaternium 37", au poly(chlorure de méthacryloyloxyéthyltriméthyl-ammonium) réticulé, en dispersion à 50% dans de l'huile minérale, et vendu sous la dénomination Salcare SC95 par la société Allied Colloids, en ce qui concerne le "Polyquaternium 32", au copolymère réticulé de l'acrylamide et du chlorure de méthacryloyloxyéthyltriméthylammonium (20/80 en poids), en dispersion à 50% dans de l'huile minérale, et vendu sous la dénomination Salcare SC92 par la société Allied Colloids, et en ce qui concerne le "Polyquaternium 35", au méthosulfate du copolymère de méthacryloyloxyéthyltriméthylammonium et de méthacryloyloxyéthyldiméthylacétylammonium, vendu sous la dénomination Plex 7525L par la société Rohm GmbH.

Les polymères substantifs du type polyammonium quaternaire utilisables selon l'invention sont les suivants :
- les polymères préparés et décrits dans le brevet français 2 270 846, constitués de motifs récurrents répondant à la formule (I) suivante : 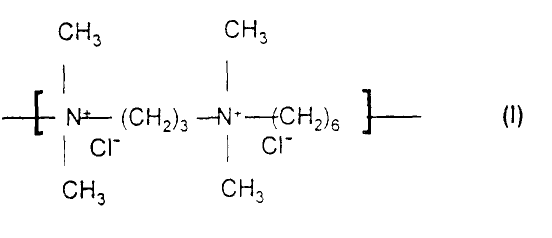 notamment ceux dont le poids moléculaire, déterminé par chromatographie par perméation de gel, est compris entre 9500 et 9900 ;
- les polymères préparés et décrits dans le brevet français 2 270 846, constitués de motifs récurrents répondant à la formule (II) suivante : 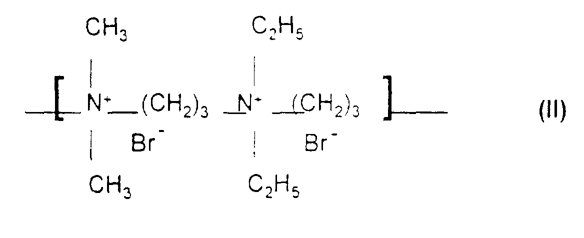 notamment ceux dont le poids moléculaire, déterminé par chromatographie par perméation de gel, est d'environ 1200 ;
- les polymères décrits et préparés dans les brevets US 4 157 388, 4 390 689, 4 702 906, 4 719 282, et constitués de motifs récurrents répondant à la formule (III) suivante : 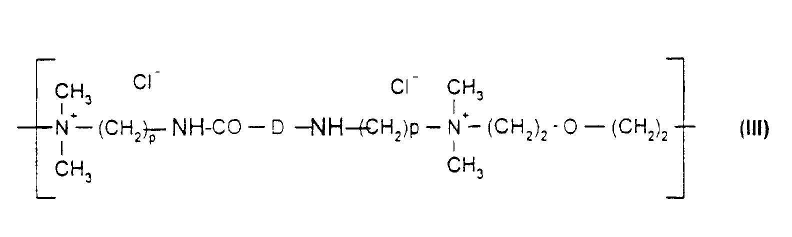 dans laquelle p désigne un nombre entier variant de 1 à 6 environ, D peut être nul ou peut représenter un groupement -(CH₂)ᵣ-CO- dans lequel r désigne un nombre égal à 4 ou à 7, et notamment ceux dont la masse moléculaire est inférieure à 100 000, de préférence inférieure ou égale à 50 000 ; de tels polymères sont notamment vendus par la société Miranol sous les dénominations "Mirapol A15", "Mirapol AD1", "Mirapol AZ1" et "Mirapol 175" ;

Parmi les polymères de Vinylpyrrolidone (PVP) à motifs cationiques utilisables conformément à l'invention, on peut citer en particulier :
a) les polymères de Vinylpyrrolidone comportant des motifs Méthacrylate de diméthylaminoéthyle : on peut citer parmi ceux-ci :
   - le copolymère Vinylpyrrolidone / Méthacrylate de diméthylaminoéthyle (20/80 en poids) vendu sous la dénomination commerciale COPOLYMER 845 par la société I.S.P.
   - les copolymères Vinylpyrrolidone / Méthacrylate de diméthylaminoéthyle quaternisés par du sulfate de diéthyle, vendus sous les dénominations GAFQUAT 734, 755, 755 S et 755 L par la société I.S.P.
   - les PVP / Méthacrylate de diméthylaminoéthyle / Polyuréthane hydrophile, vendus sous la dénomination commerciale PECOGEL GC-310 par la société U.C.I.B. ou encore sous les dénominations AQUAMERE C 1031 et C 1511 par la société BLAGDEN CHEMICALS,
   - les PVP / Méthacrylate de diméthylaminoéthyle / Oléfine en C8 à C16, quaternisés ou non quarternisés, vendus sous les dénominations GANEX ACP 1050 à 1057, 1062 à 1069, 1079 à 1086, par la société I.S.P.
   - le PVP */* Méthacrylate de diméthylaminoéthyle / Vinylcaprolactame, vendu sous la dénomination GAFFIX VC 713 par la société I.S.P.
b) les polymères de Vinylpyrrolidone comportant des motifs Méthacrylamidopropyltriméthylammonium ( M.A.P.T.A.C. ), parmi lesquels on peut citer notamment :
   - les copolymères Vinylpyrrolidone / M.A.P.T.A.C., vendus sous les dénominations commerciales GAFQUAT ACP 1011 et GAFQUAT HS 100 par la société I.S.P.
c) les polymères de Vinylpyrrolidone comportant des motifs Méthylvinylimidazolium, et parmi lesquels on peut citer plus particulièrement :
   - les PVP / Chlorure de méthylvinylimidazolium, vendus sous les dénominations LUVIQUAT FC 370, FC 550, FC 905, HM 552 par la société B.A.S.F.
   - le PVP / Chlorure de méthylvinylimidazolium / Vinylimidazole, vendu sous la dénomination LUVIQUAT 8155 par la société B.A.S.F.
   - le PVP / Méthosulfate de méthylvinylimidazolium, vendu sous la dénomination LUVIQUAT MS 370 par la société B.A.S.F.

Parmi les polysiloxanes cationiques on peut notamment citer ceux décrits dans la demande de brevet EP-A-0557203, de la page 8 ligne 48 à la page 11 ligne 9, et plus particulièrement encore les produits comprenant l'Amodiméthicone" (dénomination C.T.F.A.) de formule (IV) suivante :

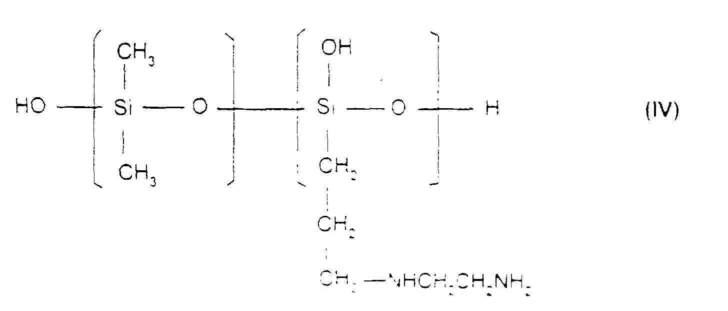

La concentration en polymère substantif cationique ou amphotère peut varier entre 0,01 et 10 % environ par rapport au poids total de la composition de teinture appliquée sur les cheveux, et de préférence entre 0,1 et 5 %.

La nature du ou des colorant(s) d'oxydation (bases d'oxydation et/ou coupleurs) utilisés dans la composition tinctoriale prête à l'emploi n'est pas critique.

Les bases d'oxydation peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (V) suivante et leurs sels d'addition avec un acide : 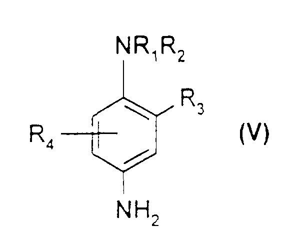 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄ monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄.
- R₄ représente un atome d'hydrogène, d'halogène ou un radical alkyle en C₁-C₄.

Parmi les groupements azotés de la formule (V) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (V) ci-dessus, on peut plus particulièrement citer la paraphénylènediamine la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphényiènediamine, la 2-β-hydroxyéthyloxy paraphénylènediamine la 2-β-acétylaminoéthyloxy paraphénylènediamine, la N-(β-méthoxyéthyl) paraphénylènediamine, et leurs sels d'addition avec un acide.

Parmi les paraphénylènediamines de formule (V) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylenediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (VI) suivante, et leurs sels d'addition avec un acide : 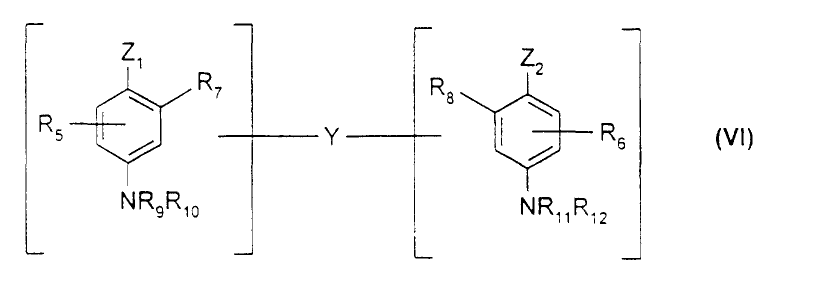 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁, et R₁₂, identiques ou différents, représentent un atome d'hydrogène, un bras de liaison Y ou un radical alkyle en C₁-C₄ ; étant entendu que les composés de formule (VI) ne comportent qu'un seul bras de liaison Y par molécule.

Parmi les groupements azotés de la formule (VI) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (VI) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (VI), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (VII) suivante, et leurs sels d'addition avec un acide : 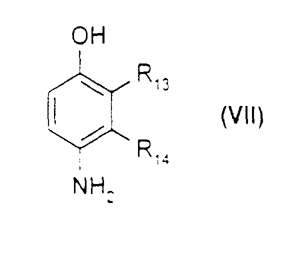 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les para-aminophénols de formule (VII) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide,

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 25,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3.4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (VIII) suivante, leurs sels d'addition avec un acide ou avec une base et leurs formes tautomères, lorsqu'il existe un équilibre tautomérique : 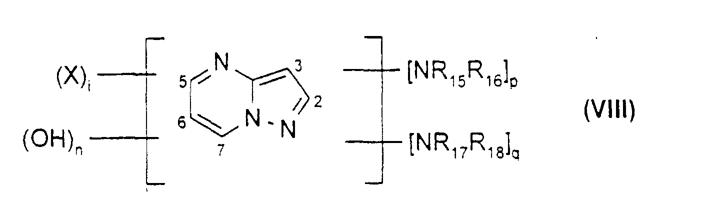 dans laquelle :
- R₁₅, R₁₆, R₁₇ et R₁₈, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C₁-C₄)alkyl- ou di-[hydroxy(C₁-C₄) alkyl]-amino alkyle en C₁-C₄; - les radicaux X désignent, identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino aikyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C4)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;

- i vaut 0, 1, 2 ou 3 ;
- p vaut 0 ou 1 ;
- q vaut 0 ou 1 ;
- n vaut 0 ou 1 ;
sous réserve que :
- la somme p + q est différente de 0,
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7) ;

Lorsque les pyrazolo-[1,5-a]-pyrimidines de formule (VIII) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

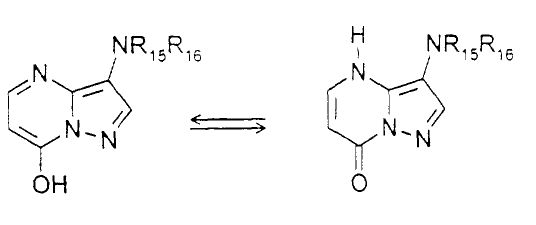

Parmi les pyrazolo-[1,5-a]-pyrimidines de formule (VIII) ci-dessus on peut notamment citer :
- la pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine,
- la 2,6-diméthyl pyrazolo-[1,5-a]-pyrimidine-3,7-diamine ;
- la 2,5 N 7. N 7-tetraméthyl pyrazolo-[1,5-a]-pyrimidine-3,7-diamine
et leurs sels d'addition et leurs formes tautomères, lorsqu'il existe un équilibre tautomérique.

Les pyrazolo-[1,5-a]-pyrimidines de formule (VIII) ci-dessus peuvent être préparées par cyclisation à partir d'un aminopyrazole selon les synthèses décrites dans les références suivantes :
- EP 628559 BEIERSDORF-LILLY
- R. Vishdu, H. Navedul, Indian J. Chem., 34b (6), 514, 1995.
- N.S. Ibrahim, K.U. Sadek, F.A. Abdel-AI, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (VIII) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977.
- E. Alcade, J. De Mendoza, J.M. Marcia-Marquina, C. Almera, J. Elguero, J. Heterocyclic Chem., 11(3), 423, 1974.
- K. Saito, I. Hori, M. Higarashi, H. Midorikawa, Bull. Chem. Soc. Japan. 47(2), 476, 1974.

La ou les bases d'oxydation conformes à l'invention représentent de préférence de 0,0005 à 12 % en poids environ du poids total de la composition tinctoriale prête à l'emploi, et encore plus préférentiellement de 0,005 à 6 % en poids environ de ce poids.

Les coupleurs utilisables sont ceux classiquement utilisés dans les compositions de teinture d'oxydation c'est-à-dire des métaphénylénèdiamines, des métaaminophénols et des métadiphénols, les dérivés mono- ou poly-hydroxylés du naphtalène, le sésamol et ses dérivés et des composés hétérocycliques tels que par exemple les dérivés indoliques, les dérivés indoliniques, les dérivés de benzimidazole, les dérivés de benzomorpholine, les dérivés de sésamol, les dérivés pyrazolo-azoliques, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoliques, les dérivés pyrazolo-[3,4-d]-thiazoliques, les dérivés S-oxyde-thiazolo-azoliques, les dérivés S,S-dioxyde-thiazolo-azoliques, et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, le 2,6-diméthyl-pyrazolo-[1,5-b]-1,2,4-triazole, le 2,6-diméthyl-[3,2-c]-1,2,4-triazole, le 6-méthyl-pyrazolo-[1,5-a]-benzimidazole, et leurs sels d'addition avec un acide.

Ces coupleurs représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

La composition tinctoriale de l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins un colorant d'oxydation tel que défini précédemment et, d'autre part, une composition (B) renfermant, dans un milieu approprié pour la teinture, au moins une enzyme de type laccase et au moins au moins un polymère substantif cationique ou amphotère puis à procéder à leur mélange au moment de l'emploi avant d'appliquer ce mélange sur les fibres kératiniques.

Selon une forme de réalisation particulière de l'invention, le polymère substantif cationique ou amphotère peut être incorporé dans la composition (A).

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Le milieu approprié pour les fibres kératiniques (ou support) des compositions tinctoriales prêtes à l'emploi conformes à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ainsi que les alcools aromatiques comme l'alcool benzylique, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH des compositions tinctoriales prêtes à l'emploi conformes à l'invention est choisi de telle manière que l'activité enzymatique de la laccase ne soit pas altérée. Il varie généralement de 4 à 11 environ, et plus préférentiellement de 6 à 9 environ.

Les compositions tinctoriales prêtes à l'emploi conformes à l'invention peuvent également renfermer divers adjuvants utilisés classiquement dans les compositions pour la teinture, tels que des agents tensio-actifs anioniques, cationiques, non-ioniques, amphotères, zwittérioniques ou leurs mélanges, des polymères, des agents épaississants, des agents antioxydants, des enzymes différentes des laccases utilisées conformément à l'invention telles que par exemple des peroxydases ou des oxydo-réductases à 2 électrons, des agents de pénétration, des agents séquestrants, des parfums, des tampons, des agents dispersants, des agents filmogènes, des agents filtrants, des vitamines, des agents conservateurs des agents opacifiants.

Bien entendu, l'homme de l'art veillera à choisir ce ou ces éventuels composés complémentaires de manière telle que les propriétés avantageuses attachées intrinsèquement aux compositions conformes à l'invention ne soient pas, ou substantiellement pas, altérées par la ou les adjonctions envisagées.

Les compositions tinctoriales prêtes à l'emploi conformes à l'invention, peuvent se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains.

Dans le cas d'une composition tinctoriale prête à l'emploi, le ou les colorants d'oxydation et la ou les laccases sont présents au sein de ladite composition qui doit être exempte d'oxygène gazeux, de manière à éviter toute oxydation prématurée du ou des colorants d'oxydation.

Des exemples concrets illustrant l'invention vont maintenant être donnés.

Dans ce qui suit ou ce qui précède, sauf mention contraire, les pourcentages sont exprimés en poids.

Les exemples suivants illustrent l'invention sans présenter un caractère limitatif.

### EXEMPLES 1 à 3 de TEINTURE

On a préparé les compositions tinctoriales prêtes à l'emploi suivante (teneurs en grammes) :

| **COMPOSITION** | **1** | **2** | **3** |
|---|---|---|---|
| Laccase issue de Rhus vernicifera à 180 unités/mg vendue par la société SIGMA | 1,8 | 1,8 | 1,8 |
| Paraphénylènediamine | 0,254 | 0,254 | 0,254 |
| 2,4-diaminophénoxyéthanol, 2HCl | 0,260 | 0,260 | 0,260 |
| Ethanol | 20 | 20 | 20 |
| Alkylpolyglucoside en solution aqueuse à 60% de matière active (M.A.) vendu sous la dénomination ORAMIX CG110 par la société SEPPIC | 4,800 | 4,800 | 4,800 |
| Polymère substantif | 1 (M.A.) | 1 (M.A.) | 1 (M.A.) |
| Agent de pH qs pH | 6,5 | 6,5 | 6,5 |
| Eau déminéralisée qsp | 100 | 100 | 100 |

Polymère substantif de la composition (1) :
   mélange polydiméthylsiloxane à groupements aminométhylaminoisobutyle / polydiméthylsiloxane, vendu sous la dénomination Q2 8220 par la société DOW CORNING.
Polymère substantif de la composition (2) :
   copolymère chlorure de diméthyldiallylammonium / acide acrylique en solution aqueuse à 40,5% vendu sous la dénomination MERQUAT 280 par la société CALGON.
Polymère substantif de la composition (3) :
   polymère de formule (I) [polycondensat tétraméthylhexaméthylènediamine / 1,3-dichloro-propylènediamine en solution aqueuse à 60%].

Les compositions tinctoriales prêtes à l'emploi décrites ci-dessus ont été appliquées à la température de 30°C sur des mèches de cheveux gris naturels à 90% de blancs pendant 40 minutes. Les cheveux ont ensuite été rincés, lavés avec un shampooing standard, puis séchés. Les cheveux ont été teints dans les trois cas en gris bleuté.

Dans les exemples décrits ci-dessus, 1,8% de Rhus vernicifera laccase à 180 unités/mg peut être remplacé par 1% de Pyricularia Orizae laccase à 100 unités/mg vendue par la société I.C.N.

## Revendications

1. Composition prête à l'emploi pour la teinture des fibres kératiniques, en particulier des fibres kératiniques humaines et plus particulièrement les cheveux humains, comprenant dans un support approprié pour les fibres kératiniques :
(a) au moins une enzyme du type laccase;
(b) au moins un polymère substantif cationique ou amphotère choisi dans le groupe formé par :
**(i)** le Polyquaternium-24 ;
**(ii)** les copolymères d'halogénure de diméthyldiallylammonium;
**(iii)** les homopolymères et copolymères d'halogénure de méthacryloyloxyéthyltriméthylammonium ;
**(iv)** les polymères polyammonium quaternaire choisis parmi ceux de formules (I), (II), et (III) suivantes :
- les polymères constitués de motifs récurrents répondant à la formule (I) suivante : 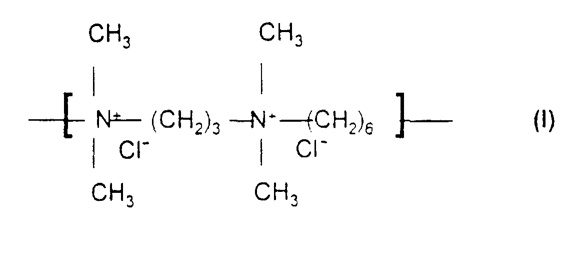
- les polymères constitués de motifs récurrents répondant à la formule (II) suivante : 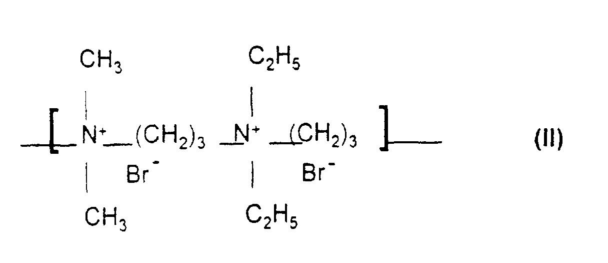
- les polymères constitués de motifs récurrents répondant à la formule (III) suivante : 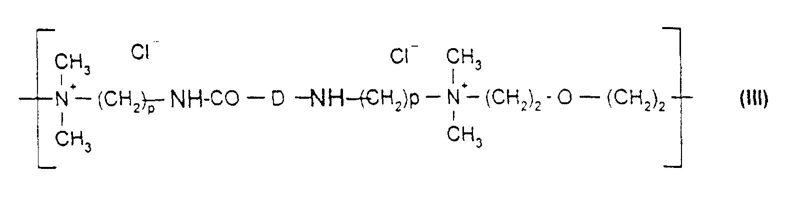 dans laquelle p désigne un nombre entier variant de 1 à 6 environ, D peut être nul ou peut représenter un groupement -(CH₂)ᵣ-CO- dans lequel r désigne un nombre égal à 4 ou à 7.
**(v)** les copolymères de vinylpyrrolidone à motifs cationiques ;
**(vi)** les polysiloxanes cationiques ;
(c) au moins un colorant d'oxydation.

2. Composition selon la revendication 1, caractérisée par le fait que la ou les laccases sont choisies parmi les laccases d'origine végétale, d'origine animale, d'origine fongique, d'origine bactérienne, ou obtenues par biotechnologie.

3. Composition selon l'une quelconque des revendications 1 à 2, où les laccases sont choisies parmi celles produites par des végétaux effectuant la synthèse chlorophyllienne.

4. Composition selon la revendication 3, où les laccases sont choisies parmi celles extraites des Anacardiacées ou des Podocarpacées, de Rosmarinus off., de Solanum tuberosum, d'Iris sp., de Coffea sp., de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.

5. Composition selon la revendication 2, où les laccases sont choisies parmi celles issues de Pyricularia orizae, de Polyporus versicolor, de Rhizoctonia praticola, de Rhus vernicifera, de Scytalidium, de Polyporus pinsitus, de Myceliophtora thermophila, de Rhizoctonia solani, de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus. de Panaeolus papilionaceus, de Panaeolus sphinctrinus, de Schizophyllum commune, de Dichomitius squalens, ainsi que leurs variantes.

6. Composition selon l'une quelconque des revendications 1 à 5, caractérisée par le fait que la ou les laccases sont présentes dans des quantités allant de 0,5 à 2000 lacu, ou de 1000 à 4.10⁷ unités u, ou de 20 à 2.10⁶ unités ulac, pour 100g de composition.

7. Composition selon la revendication 1, caractérisée par le fait que les polymères substantifs du type copolymère d'halogénure de diméthyldiallylammonium sont choisis parmi :
- le copolymère de chlorure de diallyldiméthylammonium et d'acide acrylique,
- les copolymères de chlorure de diallyldiméthylammonium et d'acrylamide.

8. Composition selon selon la revendication 1, caractérisée par le fait que les polymères substantifs du type homopolymère ou copolymère d'halogénure de méthacryloyloxyéthyltriméthylammonium sont choisis parmi :
- les homopolymères poly(chlorure de méthacryloyloxyéthyltriméthylammonium) réticulés, en dispersion à 50% dans de l'huile minérale,
- le copolymère réticulé de l'acrylamide et du chlorure de méthacryloyloxyéthyltriméthylammonium (20/80 en poids), en dispersion à 50% dans de l'huile minérale,
- le méthosulfate du copolymère de méthacryloyloxyéthyltriméthylammonium et de méthacryloyloxyéthyldiméthylacétylammonium.

9. Composition selon la revendication 1, caractérisée par le fait que les polymères substantifs du type polymère de Vinylpyrrolidone à motifs cationiques sont choisis parmi :
a) les polymères de Vinylpyrrolidone comportant des motifs Méthacrylate de diméthylaminoéthyle ;
b) les polymères de Vinylpyrrolidone comportant des motifs Méthacrylamidopropyltriméthylammonium ;
c) les polymères de Vinylpyrrolidone comportant des motifs Méthylvinylimidazolium ;

10. Composition selon la revendication 1, caractérisée par le fait que les polymères substantifs du type polysiloxane cationique sont choisis parmi les produits comprenant l'Amodiméthicone de formule (IV) suivante :

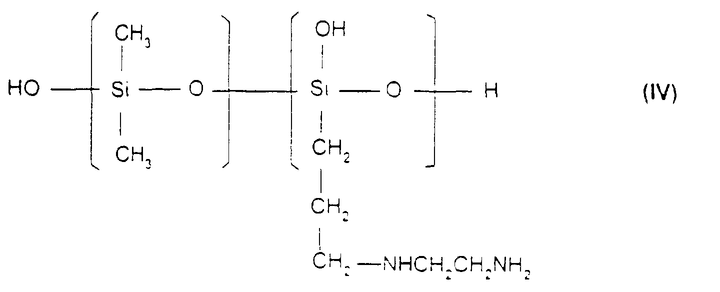

11. Composition selon l'une quelconque des revendications 1 à 10, caractérisée par le fait que la concentration en polymère substantif peut varier entre 0,01 et 10 % environ par rapport au poids total de la composition et de préférence entre 0,1 et 5 %.

12. Composition selon l'une quelconque des revendications précédentes, caractérisée par le fait que le ou les colorants d'oxydation sont choisis parmi les bases d'oxydation et/ou les coupleurs.

13. Composition selon la revendication 12, caractérisée par le fait que les bases d'oxydation sont choisis parmi les ortho- ou para- phénylènediamines, les bis-phénylalkylènediamines, les ortho- ou para- aminophénols, et les bases hétérocycliques, ainsi que les sels d'addition de ces composés avec un acide.

14. Composition selon la revendication 12 ou 13, caractérisée par le fait que les bases d'oxydation sont présentes dans des concentrations allant de 0,0005 à 12% en poids par rapport au poids total de la composition, et de préférence de 0,005 à 6%.

15. Composition selon la revendication 12, caractérisée par le fait que les coupleurs sont choisis parmi les métaphénylènediamines, les métaaminophénols, les métadiphénols, les coupleurs hétérocycliques, et les sels d'addition de ces composés avec un acide.

16. Composition selon la revendication 15, caractérisée par le fait que les coupleurs sont choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, le 2,6-diméthyl-pyrazolo-[1,5-b]-1,2,4-triazole, le 2,6-diméthyl-[3,2-c]-12,4-triazole, le 6-méthyl-pyrazolo-[1,5-a]-benzimidazole, et leurs sels d'addition avec un acide.

17. Composition selon l'une quelconque des revendications 12, 15 et 16, caractérisée par le fait que le ou les coupleurs représentent de 0,0001 à 10 % en poids du poids total de la composition tinctoriale, et de préférence de 0,005 à 5%.

18. Composition selon l'une quelconque des revendications 12 à 17, caractérisée par le fait que les sels d'addition avec un acide des bases d'oxydation et des coupleurs sont choisis parmi les chlorhydrates, les bromhydrates, les sulfates, les tartrates, les lactates et les acétates.

19. Composition selon l'une quelconque des revendications précédentes, caractérisée par le fait qu'elle contient en outre des colorants directs.

20. Composition selon l'une quelconque des revendications précédentes, caractérisée par le fait que le milieu approprié pour les fibres kératiniques (ou support) est constitué par de l'eau ou par un mélange d'eau et d'au moins un solvant organique.

21. Composition selon l'une quelconque des revendications précédentes, caractérisée par le fait que les solvants organiques peuvent être présents dans des proportions de préférence allant 1 à 40 % en poids environ par rapport au poids total de la composition, et encore plus préférentiellement allant de 5 à 30 % en poids environ.

22. Composition selon l'une quelconque des revendications précédentes, caractérisée par le fait que le pH varie de 4 à 11 environ, et de préférence de 6 à 9 environ.

23. Composition selon l'une quelconque des revendications précédentes, caractérisée par le fait qu'elle contient en plus au moins un adjuvant cosmétique utilisé classiquement dans les compositions pour la teinture des cheveux, choisi dans le groupe constitué par des agents tensio-actifs, des polymères, des agents épaississants, des agents antioxydants, des enzymes différentes des laccases utilisées conformément à l'invention telles que par exemple des peroxydases ou des oxydo-réductases à 2 électrons, des agents de pénétration, des agents séquestrants, des parfums, des tampons, des agents dispersants, des agents filmogènes, des agents filtrants, des vitamines, des agents conservateurs, des agents opacifiants,

24. Procédé de teinture des fibres kératiniques et en particulier des fibres kératiniques humaines telles que les cheveux, caractérisé par le fait qu'on applique sur lesdites fibres au moins une composition tinctoriale prête à l'emploi telle que définie dans l'une quelconque des revendications précédentes, pendant un temps suffisant pour développer la coloration désirée.

25. Procédé selon la revendication 24, caractérisé par le fait qu'il comporte une étape préliminaire consistant à stocker sous forme séparée, d'une part, une composition (A) comprenant, dans un milieu approprié pour la teinture, au moins un colorant d'oxydation tel que défini dans l'une quelconque des revendications 12 à 18 et d'autre part, une composition (B) renfermant, dans un milieu approprié pour les fibres kératiniques, au moins une enzyme du type laccase telle que définie dans l'une quelconque des revendications 1 à 6 puis à procéder à leur mélange au moment de l'emploi avant d'appliquer ce mélange sur les fibres kératiniques ; la composition (A) ou la composition (B) contenant le polymère substantif cationique ou amphotère tel que défini dans les revendications 1 et 7 à 11.

26. Dispositif à plusieurs compartiments ou "kit" de teinture, caractérisé par le fait qu'il comporte un premier compartiment renfermant la composition (A) telle que définie dans la revendication 25 et un second compartiment renfermant la composition (B) telle que définie dans la revendication 25.

## Claims

1. Ready-to-use composition for the dyeing of keratinous fibres, in particular human keratinous fibres and more particularly human hair, comprising, in a carrier appropriate for keratinous fibres:
(a) at least one enzyme of the laccase type;
(b) at least one cationic or amphoteric substantive polymer chosen from the group consisting of:
**(i)** Polyquaternium-24;
**(ii)** copolymers of dimethyldiallylammonium halide;
**(iii)** homopolymers and copolymers of methacryloyloxyethyltrimethylammonium halide;
**(iv)** poly(quaternary ammonium) polymers chosen from those of the following formulae (I), (II) and (III):
- the polymers consisting of recurring units corresponding to the following formula (I): 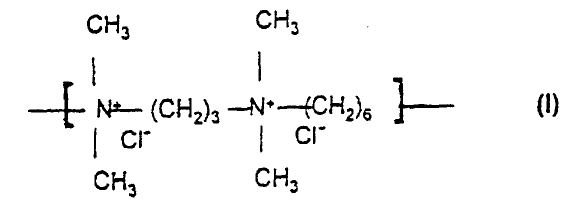
- the polymers consisting of recurring units corresponding to the following formula (II): 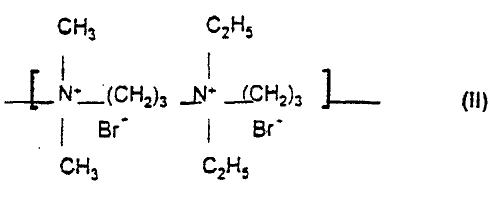
- the polymers consisting of recurring units corresponding to the following formula (III): 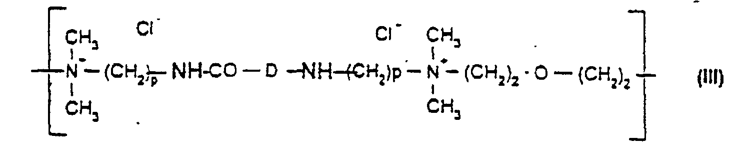 in which p denotes an integer varying from 1 to 6 approximately, D may be zero or may represent a group -(CH₂)ᵣ-CO- in which r denotes a number equal to 4 or to 7;
**(v)** copolymers of vinylpyrrolidone containing cationic units;
**(vi)** cationic polysiloxanes;
(c) at least one oxidation dye.

2. Composition according to Claim 1, characterized in that the laccase(s) are chosen from laccases of plant origin, animal origin, fungal origin, bacterial origin or are obtained by biotechnology.

3. Composition according to either of Claims 1 to 2, where the laccases are chosen from those produced by plants performing chlorophyll synthesis.

4. Composition according to Claim 3, where the laccases are chosen from those extracted from Anacardiaceae or Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Indian pipe), Aesculus sp., Acer pseudoplatanus, Prunus persica, Pistacia palaestina.

5. Composition according to Claim 2, where the laccases are chosen from those derived from Pyricularia orizae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitius squalens and variants thereof.

6. Composition according to any one of Claims 1 to 5, characterized in that the laccase(s) are provided in quantities ranging from 0.5 to 2000 lacu, or from 1000 to 4×10⁷, or from 20 to 2×10⁶ lacu units, per 100 g of composition.

7. Composition according to Claim 1, characterized in that the substantive polymers of the copolymer of dimethyldiallylammonium halide type are chosen from:
- the copolymer of diallyldimethylammonium chloride and of acrylic acid.
- the copolymers of diallyldimethylammonium chloride and of acrylamide.

8. Composition according to Claim 1, characterized in that the substantive polymers of the homopolymer or copolymer of methacryloyloxyethyltrimethylammonium halide type are chosen from:
- the crosslinked poly(methacryloyloxyethyltrimethylammonium chloride) homopolymers as a 50% dispersion in mineral oil,
- the crosslinked copolymer of acrylamide and of methacryloyloxyethyltrimethylammonium chloride (20/80 by weight) as a 50% dispersion in mineral oil,
- the methosulphate of the copolymer of methylacryloyloxyethyltrimethylammonium and of methacryloyloxyethyldimethylacetylammonium.

9. Composition according to Claim 1, characterized in that the substantive polymers of the polymer of vinylpyrrolidone containing cationic units type are chosen from:
a) the polymers of vinylpyrrolidone comprising dimethylaminoethyl methacrylate units;
b) the polymers of vinylpyrrolidone comprising methacrylamidopropyltrimethylammonium units;
c) the polymers of vinylpyrrolidone comprising methylvinylimidazolium units.

10. Composition according to Claim 1, characterized in that the substantive polymers of the cationic polysiloxane type are chosen from the products comprising amodimethicone of the following formula (IV):

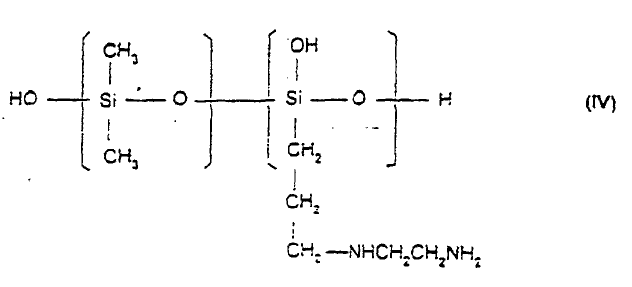

11. Composition according to any one of Claims 1 to 10, characterized in that the concentration of substantive polymer may vary between 0.01 and 10% approximately relative to the total weight of the composition and preferably between 0.1 and 5%.

12. Composition according to any one of the preceding claims, characterized in that the oxidation dye(s) are chosen from oxidation bases and/or couplers.

13. Composition according to Claim 12, characterized in that the oxidation bases are chosen from ortho- or para-phenylenediamines, bis-phenylalkylenediamines, ortho- or para-aminophenols, and heterocyclic bases, as well as the addition salts of these compounds with an acid.

14. Composition according to Claim 12 or 13, characterized in that the oxidation bases are present in concentrations ranging from 0.0005 to 12% by weight relative to the total weight of the composition, and preferably from 0.005 to 6%.

15. Composition according to Claim 12, characterized in that the couplers are chosen from meta-phenylenediamines, meta-aminophenols, meta-diphenols, heterocyclic couplers, and the addition salts of these compounds with an acid.

16. Composition according to Claim 15, characterized in that the couplers are chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 6-hydroxyindole, 4-hydroxyindole, 4-hydroxy-N-methylindole, 6-hydroxyindoline, 2,6-dihydroxy-4-methylpyridine, 1-H-3-methylpyrazol-5-one, 1-phenyl-3-methylpyrazol-5-one, 2,6-dimethyl-pyrazolo[1,5-b]-1,2,4-triazole, 2,6-dimethyl[3,2-c]-1,2,4-triazole, 6-methylpyrazolo[1,5-a]benzimidazole, and their addition salts with an acid.

17. Composition according to any one of Claims 12, 15 and 16, characterized in that the coupler(s) represent from 0.0001 to 10% by weight of the total weight of the dyeing composition, and preferably from 0.005 to 5%.

18. Composition according to any one of Claims 12 to 17, characterized in that the addition salts with an acid of the oxidation bases and couplers are chosen from hydrochlorides, hydrobromides, sulphates, tartrates, lactates and acetates.

19. Composition according to any one of the preceding claims, characterized in that it contains in addition direct dyes.

20. Composition according to any one of the preceding claims, characterized in that the medium appropriate for keratinous fibres (or carrier) consists of water or of a mixture of water and at least one organic solvent.

21. Composition according to any one of the preceding claims, characterized in that the organic solvents can be present in proportions preferably ranging from 1 to 40% by weight approximately relative to the total weight of the composition, and still more preferably ranging from 5 to 30% by weight approximately.

22. Composition according to any one of the preceding claims, characterized in that the pH varies from 4 to 11 approximately, and preferably from 6 to 9 approximately.

23. Composition according to any one of the preceding claims, characterized in that it contains in addition at least one cosmetic adjuvant conventionally used in hair dyeing compositions, chosen from the group consisting of surfactants, polymers, thickeners, antioxidants, enzymes different from the laccases used in accordance with the invention, such as for example peroxidases or oxidoreductases containing 2 electrons, penetrating agents, sequestering agents, perfumes, buffers, dispersing agents, film-forming agents, screening agents, vitamins, preservatives or opacifying agents.

24. Method of dyeing keratinous fibres, and in particular human keratinous fibres such as hair, characterized in that at least one ready-to-use dyeing composition as defined in any one of the preceding claims is applied to the said fibres for a sufficient time to develop the desired colour.

25. Method according to Claim 24, characterized in that it comprises a preliminary step consisting in storing in a separate form, on the one hand, a composition (A) comprising, in a medium appropriate for dyeing, at least one oxidation dye as defined in any one of Claims 12 to 18 and on the other hand, a composition (B) containing, in a medium appropriate for keratinous fibres, at least one enzyme of the laccase type as defined in any one of Claims 1 to 6, and then in mixing them at the time of use before applying this mixture to the keratinous fibres; the composition (A) or the composition (B) containing the cationic or amphoteric substantive polymer as defined in Claims 1 and 7 to 11.

26. Multicompartment device or dyeing "kit", characterized in that it comprises a first compartment containing the composition (A) as defined in Claim 25 and a second compartment containing the composition (B) as defined in Claim 25.

## Patentansprüche

1. Gebrauchsfertige Zusammensetzung zum Färben von Keratinfasern, insbesondere menschlichen Keratinfasern und ganz besonders des Haares, die in einem für Keratinfasern geeigneten Medium enthält:
(a) mindestens ein Enzym vom Laccase-Typ,
(b) mindestens ein kationisches oder amphoteres substantives Polymer, das ausgewählt ist unter:
(i) Polyquaternium-24;
(ii) Dimethyldiallylammoniumhalogenid-Copolymeren;
(iii) Methacryloyloxyethyltrimethylammoniumhalogenid-Homopolymeren und Methacryloyloxyethyltrimethylammoniumhalogenid-Copolymeren;
(iii) quartären Polyammoniumpolymeren, die unter den folgenden Formeln (I), (II) und (III) ausgewählt sind:
- Polymeren, die aus wiederkehrenden Einheiten der folgenden Formel (I) bestehen: 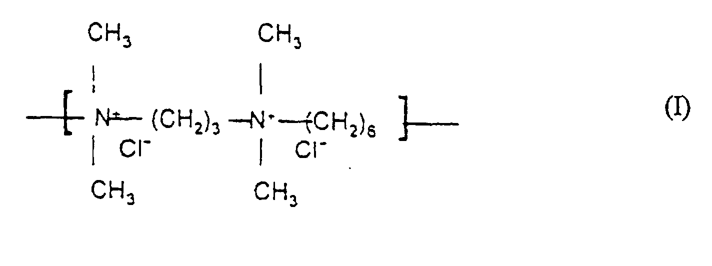
- Polymeren, die aus wiederkehrenden Einheiten der folgenden Formel (II) bestehen: 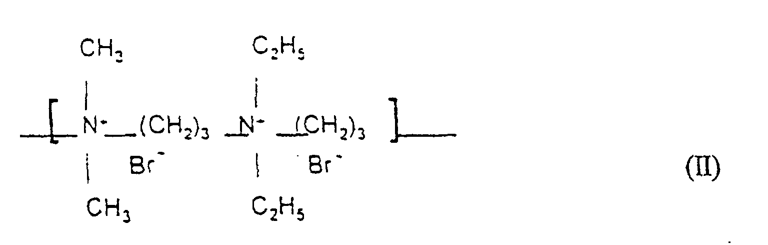
- Polymeren, die aus wiederkehrenden Einheiten der folgenden Formel (III) bestehen: 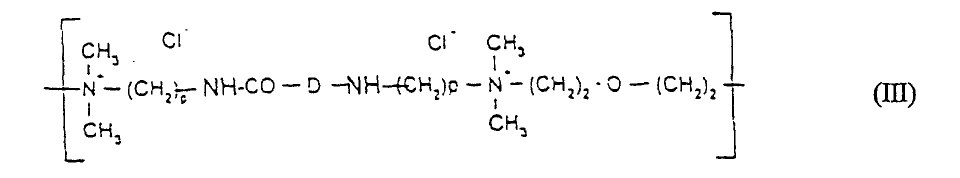 worin p eine ganze Zahl von etwa 1 bis 6 bedeutet und die Gruppe D nicht vorhanden sein kann oder eine Gruppe -(CH₂)ᵣ-CO ist, wobei r 4 oder 7 bedeutet,
(v) Vinylpyrrolidoncopolymeren mit kationischen Einheiten; und
(vi) kationischen Polysiloxanen, und
(c) mindestens einen Oxidationsfarbstoff.

2. Zusammensetzung nach Anspruch 1, dadurch gekennzeichnet, daß die Laccase(n) unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt sind.

3. Zusammensetzung nach einem der Ansprüche 1 oder 2, wobei die Laccasen unter den Laccasen ausgewählt sind, die von Pflanzen gebildet werden, die Chloropyll synthetisieren.

4. Zusammensetzung nach Anspruch 3, wobei die Laccasen unter den Laccasen ausgewählt sind, die aus Anacardiaceae oder Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica oder Pistacia palaestina extrahiert wurden.

5. Zusammensetzung nach Anspruch 2, wobei die Laccasen unter den Laccasen ausgewählt sind, die von Pyricularia oryzae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

6. Zusammensetzung nach einem der Ansprüche 1 bis 5, dadurch gekennzeichnet, daß die Laccase(n) in einem Mengenanteil von Bereich von 0,5 bis 2000 lacu oder 1000 bis 4·10⁷ Einheiten u oder 20 bis 2·10⁶ Einheiten ulac pro 100 g Zusammensetzung vorliegen.

7. Zusammensetzung nach Anspruch 1, dadurch gekennzeichnet, daß die substantiven Polymere vom Typ der Dimethyldiallylammoniumhalogenid-Copolymere ausgewählt sind unter:
- dem Copolymer von Diallyldimethylammoniumchlorid und Acrylsäure; und
- den Copolymeren von Dimethyldiallylammoniumchlorid und Acrylamid.

8. Zusammensetzung nach Anspruch 1, dadurch gekennzeichnet, daß die substantiven Polymere vom Typ Methacryloyloxyethyltrimethylammoniumhalogenid-Homopolymer oder -Copolymer ausgewählt sind unter:
- vernetzten Poly(methacryloyloxyethyltrimethylammoniumchlorid)-Homopolymeren, 50%ige Dispersion in Mineralöl;
- dem vernetzten Copolymer von Acrylamid und Methacryloyloxyethyltrimethylammoniumchlorid (20/80 Gew.-%), 50%ige Dispersion in Mineralöl;
- dem Methacryloyloxyethyltrimethylammonium/Methacryloyloxyethyldimethylacetylammoniummethosulfat-Copolymer.

9. Zusammensetzung nach Anspruch 1, dadurch gekennzeichnet, daß die substantiven Polymere vom Typ der Vinylpyrrolidonpolymere mit kationischen Einheiten ausgewählt sind unter:
a) den Vinylpyrrolidonpolymeren mit Dimethylaminoethylmethacrylateinheiten;
b) den Vinylpyrrolidonpolymeren mit Methacrylamidopropyltrimethylammoniumeinheiten; und
c) den Vinylpyrrolidonpolymeren mit Methylvinylimidazoliumeinheiten.

10. Zusammensetzung nach Anspruch 1, dadurch gekennzeichnet, daß die substantiven Polymere vom Typ der kationischen Polysiloxane unter den Produkten ausgewählt sind, die das "Amodimethicon" der folgenden Formel (IV) enthalten:

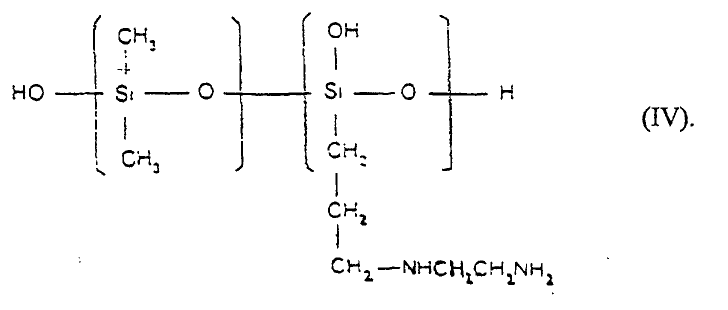

11. Zusammensetzung nach einem der Ansprüche 1 bis 10, dadurch gekennzeichnet, daß die Konzentration der substantiven Polymere im Bereich von etwa 0,01 bis 10 %, bezogen auf das Gesamtgewicht der Zusammensetzung, und vorzugsweise im Bereich von 0,1 bis 5 % liegen kann.

12. Zusammensetzung nach einem der vorhergehenden Ansprüche, dadurch gekennzeichnet, daß der oder die Oxidationsfarbstoffe unter den Oxidationsbasen und/oder den Kupplern ausgewählt sind.

13. Zusammensetzung nach Anspruch 12, dadurch gekennzeichnet, daß die Oxidationsbasen unter den o- oder p-Phenylendiaminen, Bisphenylalkylendiaminen, o- oder p-Aminophenolen und den heterocyclischen Basen sowie den Additionssalzen dieser Verbindungen mit einer Säure ausgewählt sind.

14. Zusammensetzung nach Anspruch 12 oder 13, dadurch gekennzeichnet, daß die Oxidationsbasen in Konzentrationen von 0,0005 bis 12 Gew.-%, bezogen auf das Gesamtgewicht der Zusammensetzung, und vorzugsweise 0,005 bis 6 Gew.-% vorliegen.

15. Zusammensetzung nach Anspruch 12, dadurch gekennzeichnet, daß die Kuppler unter den m-Phenylendiaminen, m-Aminophenolen, m-Dihydroxybenzolen und heterocyclischen Kupplern und deren Additionssalzen mit einer Säure ausgewählt sind.

16. Zusammensetzung nach Anspruch 15, dadurch gekennzeichnet, daß die Kuppler unter 2-Methyl-5-amino-phenol, 5-N-(β-Hydroxyethyl)-amino-2-methyl-phenol, 3-Amino-phenol, 1,3-Dihydroxy-benzol, 1,3-Dihydroxy-2-methyl-benzol, 4-Chlor-1,3-dihydroxy-benzol, 2,4-Diamino-1-(β-hydroxyethyloxy)-benzol, 2-Amino-4-(β-hydroxyethylamino)-1-methoxy-benzol, 1,3-Diamino-benzol, 1,3-Bis(2,4-diaminophenoxy)-propan, Sesamol, α-Naphthol, 6-Hydroxyindol, 4-Hydroxyindol, 4-Hydroxy-N-methyl-indol, 6-Hydroxyindolin, 2,6-Dihydroxy-4-methyl-pyridin, 1-H-3-Methyl-pyrazol-5-on, 1-Phenyl-3-methyl-pyrazol-5-on, 2,6-Dimethylpyrazolo-[1,5-b]-1,2,4-triazol, 2,6-Dimethyl-[3,2-c]-1,2,4-triazol, 6-Methylpyrazolo-[1,5-a]-benzimidazol und den Additionssalzen dieser Verbindungen mit einer Säure ausgewählt sind.

17. Zusammensetzung nach einem der Ansprüche 12, 15 und 16, dadurch gekennzeichnet, daß die Kuppler 0,0001 bis 10 Gew.-% des Gesamtgewichts der Färbemittelzusammensetzung und vorzugsweise 0,005 bis 5 Gew.-% ausmachen.

18. Zusammensetzung nach einem der Ansprüche 12 bis 17, dadurch gekennzeichnet, daß die Additionssalze der Oxidationsbasen und Kuppler mit einer Säure unter den Hydrochloriden, Hydrobromiden, Sulfaten, Tartraten, Lactaten und Acetaten ausgewählt sind.

19. Zusammensetzung nach einem der vorhergehenden Ansprüche, dadurch gekennzeichnet, daß sie ferner Direktfarbstoffe enthält.

20. Zusammensetzung nach einem der vorhergehenden Ansprüche, dadurch gekennzeichnet, daß das für Keratinfasern geeignete Medium (oder der Träger) aus Wasser oder einem Gemisch von Wasser und mindestens einem organischen Lösungsmittel besteht.

21. Zusammensetzung nach einem der vorhergehenden Ansprüche, dadurch gekennzeichnet, daß die organischen Lösungsmittel in Mengenanteilen vorzugsweise im Beriech von etwa 1 bis 40 Gew.-%, bezogen auf das Gesamtgewicht der Zusammensetzung, und noch bevorzugter etwa 5 bis 30 Gew.-% vorliegen können.

22. Zusammensetzung nach einem der vorhergehenden Ansprüche, dadurch gekennzeichnet, daß der pH-Wert im Bereich von etwa 4 bis 11 und vorzugsweise etwa 6 bis 9 liegt.

23. Zusammensetzung nach einem der vorhergehenden Ansprüche, dadurch gekennzeichnet, daß sie ferner mindestens einen kosmetischen Zusatzstoff enthält, der herkömmlich in Zusammensetzungen zum Färben der Haare verwendet wird und der unter den grenzflächenaktiven Stoffen, Polymeren, Verdickungsmitteln, Antioxidantien, Enzymen, die von den erfindungsgemäß verwendeten Laccasen verschieden sind, beispielsweise Peroxydasen und Oxido-reductasen mit 2 Elektronen, Penetrationsmitteln, Maskierungsmitteln, Parfums, Puffern, Dispergiermitteln, Filmbildnern, Filtern, Vitaminen, Konservierungsmitteln und Trübungsmitteln ausgewählt ist.

24. Verfahren zum Färben von Keratinfasern und insbesondere menschlichen Keratinfasern, wie dem Haar, dadurch gekennzeichnet, daß auf die Fasern mindestens eine gebrauchsfertige Färbemittelzusammensetzung nach einem der vorhergehenden Ansprüche während einer Zeitspanne, die zur Entwicklung der gewünschten Färbung ausreichend ist, aufgebracht wird.

25. Verfahren nach Anspruch 24, dadurch gekennzeichnet, daß es einen vorbereitenden Schritt umfasst, der darin besteht, einerseits eine Zusammensetzung (A), die in einem zum Färben geeigneten Medium mindestens einen Oxidationsfarbstoff nach einem der Ansprüche 12 bis 18 enthält, und andererseits eine Zusammensetzung (B) getrennt voneinander aufzubewahren, die in einem für Keratinfasern geeigneten Medium mindestens ein Enzym vom Laccase-Typ nach einem der Ansprüche 1 bis 6 enthält, und diese bei der Anwendung zu vermischen, bevor das Gemisch auf die Keratinfasern aufgebracht wird, wobei die Zusammensetzung (A) oder die Zusammensetzung das kationische oder amphotere substantive Polymer nach einem der Ansprüche 1 und 7 bis 11 enthält.

26. Vorrichtung mit mehreren Abteilungen oder "Kit" zum Färben, dadurch gekennzeichnet, daß sie eine erste Abteilung mit der in Anspruch 25 definierten Zusammensetzung (A) und eine zweite Abteilung mit der in Anspruch 25 definierten Zusammensetzung (B) enthält.
